# EUROPEAN PATENT APPLICATION

(11) **EP 1 574 220 A1**
(43) Date of publication of application: **14.09.2005**
(21) Application number: 03780782.3
(22) Date of filing: 16.12.2003
(51) Int. Cl.: A61K 47/02, A61K 47/12, A61K 47/38, A61K 9/16, A61K 9/20, A61K 9/48, A61K 31/4422

(54) **LIGHT-BLOCKING AGENT AND FILM-FORMING COMPOSITION**

(30) Priority: 17.12.2002 JP 2002365323; 27.06.2003 JP 2003185232
(71) Applicant: WAKUNAGA PHARMACEUTICAL CO., LTD., Osaka 532-0003 (JP)
(72) Inventor: SAKATA, Yuko, c/o Wakunaga Pharmaceutical Co, Ltd, Takata-gun, Hiroshima 739-1195 (JP); ICHIKAWA, Makoto, Wakunaga Pharmaceutical Co, Ltd, Takata-gun, Hiroshima 739-1195 (JP); SHIRAISHI, Sumihiro, Koda-cho, Takata-gun, Hiroshima 739-1195 (JP)
(74) Representative: Andrae, Steffen
(86) International application number: PCT/JP2003/016100
(87) International publication number: WO 2004/054619

(57) **Abstract**

A light-shielding agent and a film-forming composition containing it, which prevent separation of formulation surfaces or powder floating phenomenon, which are caused by irradiation by light or ultraviolet light, improve luster of formulation surfaces, maintain external appearance of formulations, and do not impair stability or quality of pharmaceutical preparations, or the like, are provided. The light-shielding agent includes a calcium-containing compound, and the film-forming composition includes the light-shielding agent including the calcium-containing compound and a film-forming base agent. The present invention also provides a formulation and a capsule, which are coated with both or either of the above-mentioned light-shielding agent and/or the above-mentioned film-forming composition.

## Description

### TECHNICAL FIELD

This invention relates to a novel light-shielding agent and a film-forming composition, which can suppress separation and powder floating phenomena generated on surfaces of formulations by irradiation of light or ultraviolet light, and which can maintain stability and quality of pharmaceutical preparations or the like, and relates to a formulation and a capsule using the same.

This application claims priority on Japanese Patent Applications Nos. 2002-365323 and 2003-185232, whose descriptions are incorporated herein by reference.

### BACKGROUND ART

Some pharmaceutical preparations, health foods, foods, and cosmetic preparations contain formulation ingredients, which are unstable when exposed to light, oxygen, or moisture (humidity), or which have unpleasant odors, bitter taste, or stimulators. Therefore, these problems have been attacked by coating the items with film-forming compositions containing light-shielding agents in order to ensure their stability or to mask them.

Although various materials have been used as the light-shielding agents, titanium oxides have been most frequently used for a long time in pharmaceutical and cosmetic fields because of their high safety. However, they have problems in that they may not maintain the quality of products, because they may damage the stability of some ingredients formulated as drugs or the like, or may promote decomposition of the ingredients by producing free radicals when irradiated by ultraviolet light. Moreover, when formulations coated with film-forming compositions containing titanium oxides are irradiated by (ultraviolet) light, separation of their coating films or powder floating phenomena may be sequentially caused. As a result, it may be difficult to maintain coating conditions, which may damage external appearance, or may promote decomposition of their ingredients caused by light, oxygen, or moisture, as a result of which the coated items may not sufficiently exert the effects expected of them as pharmaceutical preparations or the like.

In order to solve these problems, various attempts have been made, so that, for example, a pharmaceutical preparation coated with a film-foaming agent, containing a light-shielding agent (such as titanium oxide or the like) which produces free radicals under irradiation by ultraviolet light, and a radical eliminating agent, has been studied (see, for example, Japanese Patent Application, First Publication No. Hei 11-147819). Moreover, a formulation including a drug coated with a film-foaming agent containing talc and/or barium sulfate (see, for example, Japanese Patent Application, First Publication No. 2002-212104) has also been studied.

However, these attempts have not yet solved the aforementioned problems. Moreover, it is complicated to prepare these film-foaming agents, because their bases such as titanium oxide or the like are insoluble in water and organic solvents (which causes heterogeneity of their solutions following preparation of fine particle suspensions and precipitation of their fine particles). Moreover, because the suspensions are sprayed, they have problems such as that they cannot prevent occurrences of bumpy conditions on surfaces of tablets and decrease in strength of coated films, or that they may cause trouble during coating by plugging spray nozzles, or by depositting solid materials at tips of the nozzles, which may cause liquid dropping, or the like. Therefore, novel light-shielding agents and film-forming compositions are required to be developed.

### DISCLOSURE OF INVENTION

Objects of the present invention are to provide a light-shielding agent and a film-forming composition, which can prevent separation and powder floating phenomena generated on surfaces of formulations by irradiation of light or ultraviolet light, and which can improve luster of the formulation surfaces, maintain their external appearance for a long time, and maintain, for a long time, stability or quality of pharmaceutical preparations, or the like, and to provide formulations and capsules using the same.

As a result of diligent study, the present inventors found that use of a calcium-containing compound as a novel light-shielding agent enables production of formulations which can maintain, for a long time, stability and quality of pharmaceutical preparations, or the like, and which prevent separation of the surface or powder floating phenomena, which are caused by irradiation by light or ultraviolet light. Moreover, they found that a film-forming composition characterized by including the calcium-containing compound and a film-forming base agent can also exert effects similar to the aforementioned effects.

In this specification, "calcium-containing compound" refers to a compound containing at least one of calcium salts, calcium hydrates, calcium oxides, calcium complexes such as dolomite, hydroxyapatite, and the like, and other compounds including calcium.

That is, the first aspect of the present invention provides a light-shielding agent comprising a calcium-containing compound.

The second aspect of the present invention provides a film-forming composition comprising the calcium-containing compound and a film-forming base agent.

The third aspect of the present invention provides a formulation coated with the aforementioned light-shielding agent comprising the calcium-containing compound or the aforementioned film-forming composition.

The fourth aspect of the present invention provides a capsule comprising the film-forming composition according to the second aspect.

### BEST MODE FOR CARRYING OUT THE INVENTION

The light-shielding agent according to the present invention contains at least one calcium-containing compound. Examples of the calcium-containing compounds used in the present invention include calcium salts, calcium hydrates, calcium oxides, calcium complexes (for example, dolomite (CaMg(CO₃)₂) or hydroxyapatite (Ca₁₀(PO₄)₆(OH)₂)), and the like. In the light-shielding agent and the film-forming composition according to the present invention, compositions including calcium-containing compounds originating from minerals, such as Longgu (Longgu is a fossil of bones of ancient megavertebrates and is used as a crude drug), gypsum (which is a natural hydrous calcium sulfate, and is used as a crude drug), or the like, compositions including calcium-containing compounds originating from natural products, such as oyster shells (oyster shells, which are shells of Oyster Osteria gigas Thunberg of Ostrea denselamellosa family (Osteridae), and which is used as a crude drug), coral calcium (originating from coral), cow bone powders, fish bone powders, shell powders, egg shell powders, or the like, or calcinated products of these compositions may be directly included, or may be included after being purified.

It is preferable to use at least one selected from the group consisting of calcium salts, calcium hydrates, and calcium oxides, particularly calcium salts, as the calcium-containing compound used in the present invention.

Although both inorganic calcium salts and organic calcium salts can be used as the calcium salts used in the present invention, and there are no particular limitations imposed on them, provided that they can be substantially dissolved in water, organic solvents, mixtures of water and organic solvents (particularly, alcohols), or solutions of film-forming base agents added to these solvents, water-soluble calcium salts are more preferable.

Although examples of the inorganic calcium salts include calcium fluoride, calcium chloride, calcium bromide, calcium carbonate, calcium bicarbonate, calcium phosphate, calcium hydrogenphosphate, calcium monohydrogen phosphate, calcium dihydrogen phosphate, calcium silicate, calcium sulfate, calcium hydrogensulfate, calcium nitrate, and the like, calcium chloride, calcium phosphate, calcium sulfate, and calcium nitrate are preferable, and calcium chloride is particularly preferable. Although there are no particular limitations imposed on quality of the inorganic calcium salts, purity thereof is preferably 70% or more, more preferably 80% or more, and particularly preferably 90% or more.

Although examples of the organic calcium salts include calcium acetate, calcium citrate, calcium tartrate, calcium pantothenate, calcium gluconate, calcium succinate, calcium glycerophosphate, calcium saccharate, calcium stearate, calcium ascorbate, calcium lactate, and the like, calcium lactate and calcium gluconate are particularly preferable. In this specification, "saccharic acids" refers to carboxylic acids produced by formally oxidizing aldose groups to aldehyde groups, and "calcium saccharates" refers to calcium salts of the saccharic acids. Although there are no particular limitations imposed on quality of the organic calcium salts, purity thereof is preferably 70% or more, more preferably 80% or more, and particularly preferably 90% or more.

The film-forming composition of the second aspect of the present invention contains at least one of the calcium-containing compounds and at least one film-forming base agent. The film-forming composition of the second aspect of the present invention has merits such that it can inhibit a separating of a coating layer from the surface of a formulation or can prevent powder floating phenomenon, while maintaining general properties necessary for the film-forming composition, such as excellent film formability, abilities for forming tough and flexible films, harmlessness to the human body, smell-less properties, chemical inactivity, and the like. Moreover, the film-forming composition according to the present invention can be easily prepared, and can prevent trouble during coating steps, and has excellent workability during coating, because it is different from the conventional film-forming composition containing titanium oxide, in that it is not a suspension but a solution, when it is added to a solvent, and both of the calcium-containing compound and the film-forming base agent are dissolved in a solvent of the same nature (for example, both of the calcium-containing compound and the film-forming base agent are water-soluble). Moreover, it can form white films having excellent luster without being supplemented with additives.

Although there are no particular limitations imposed on the film-forming base agent used in the present invention, examples thereof include generally known agents, for example, 1) water-soluble film-forming base agents: cellulose base polymers such as methyl cellulose, ethyl cellulose, methylhydroxyethyl cellulose, hydroxypropyl cellulose, hydroxypropylmethyl cellulose, and the like, synthetic polymers such as polyvinylacetal diethylamino acetate, aminoalkylmethacrylate copolymer E (Eudragit-E (commercial name), Rohm Pharma Co. Ltd.), polyvinylpyrolidone, and the like, polysaccharides such as pullulan, carrageenan (polymeric polysaccharides used as gelling agents that provide viscosity and are extracted from seaweeds (order Gigartinales)), hemilose (polysaccharides contained in plants), alginic acids, mannitols, and the like, disaccharides such as saccharose and the like, monosaccharides such as glucose, and the like, sugar alcohols such as sorbitol, and the like, gelatin, 2) sustained-release film-forming base agents: cellulose base polymers such as methyl cellulose, ethyl cellulose, and the like, acrylate base polymers such as ethyl acrylate-methyl methacrylate copolymer suspensions (Eudragit NE (commercial name), Rohm Pharma Co. Ltd.), and the like, 3) enteric film-forming base agents: cellulose base polymers such as hydroxypropylmethyl cellulose phthalate, hydroxypropylmethyl cellulose acetate succinate, carboxymethylethyl cellulose, and the like, acrylate base polymers such as methacrylate copolymer L (Eudragit L (commercial name), Rohm Pharma Co. Ltd.), methacrylate copolymer LD (Eudragit L-30D55 (commercial name), Rohm Pharma Co. Ltd.), and the like, and these may be used together. Among these, cellulose base polymers are preferably used, and methylhydroxyethyl cellulose, hydroxypropyl cellulose, and hydroxypropylmethyl cellulose are more preferably used.

In the present invention, "saccharides" refers to the generic name of monosaccharides, disaccharides, and polysaccharides, of carbohydrates, and polysaccharides including cellulose base polymers.

The content of the calcium salts and the like in the film-forming composition is preferably 0.1 to 150% by weight, more preferably 0.5 to 100% by weight, and even more preferably 1 to 75% by weight, relative to the weight of the film-forming base agent. When the content is below 0.1 % by weight, effects of contained calcium and the like may not be sufficiently exerted, and when the content is over 150% by weight, the content of calcium and the like is too much to sufficiently exert synergistic effects with bases.

Although the film-forming composition according to the present invention substantially includes the light-shielding agent and the film-forming base agent, it may include, as needed, various additives, for example, plasticizers: polyethylene glycol, propylene glycol, glycerins, triacetin, middle chain fatty acid triglyceride, acetylglycerin fatty acid esters, triethyl citrate, or the like, flavors: lemon oil, oranges, dl- or 1-menthol, or the like, or other conventionally used additives.

By using the light-shielding agent or the film-forming composition according to the present invention to coat formulations, it is possible to inhibit separation of the surface of the formulations or powder floating phenomenon, which are caused by irradiation by light or ultraviolet light, to improve luster of the surface of the formulations, to maintain their external appearance for a long time, to produce light-shielding agents and film-forming compositions which can maintain stability and quality of pharmaceutical preparations, or the like, for a long time, and to produce formulations and capsules including them. To these, coloring agents may be added, as needed, and examples of the coloring agents include generally known coloring agents, for example, iron oxide, β-carotin, chlorophyll, water-soluble edible tar dyes, yellow No.4 aluminium lake, and the like.

In the present invention, radical eliminating agents may be optionally added to the film-forming composition, as needed. Although the radical eliminating agents which may be used are not particularly limited, generally known radical eliminating agents may be used. For example, β-carotin, vitamin Cs such as sodium ascorbate, calcium ascorbate, and the like, vitamin Es such as d-α-tocopherol, dl-α-tocopherol acetate, and the like, sulphites such as sodium hydrogen sulphite, potassium hydrogen sulphite, sodium sulphite, potassium sulphite, sodium metabisulfite, and the like, amino acids such as cysteine and the like may be used, and these may be used together.

The novel light-shielding agents according to the first aspect of the present invention may be in the form of powders, granules, solids, suspensions, solutions, or the like.

The film-forming compositions according to the second aspect of the present invention may be in the form of powders, granules, solids, suspensions, solutions, or the like. Although methods of producing the film-forming compositions are not particularly limited, they may be produced according to generally known methods. For example, the calcium-containing compound and the film-forming base agent are mixed, after the additives are added, as needed, or are added to a solvent, followed by removing the solvent therefrom, if needed, to produce the film-forming compositions. Although examples of the solvent used for the preparation include water, and organic solvents such as alcohols such as methyl alcohol, ethyl alcohol, and the like, ethers such as dimethyl ether, diethyl ether, and the like, ketones such as acetone, and the like, and mixtures thereof, water, methyl alcohol, ethyl alcohol, and mixtures of these are preferably used.

Although methods of coating formulations with the light-shielding agent or the film-forming composition according to the present invention are not particularly limited, they may be coated according to generally known methods. For example, a spray coating method, a pan coating method, a rolling method, or the like may be used, and the spray coating method is particularly preferable. In this specification, the spray coating method is a method by which, for example, the calcium-containing compound, the film-forming base agent, and the like are dissolved in water to produce a coating solution, which is then sprayed and dried on tablets or granules (by using coating machines such as high coaters, flow coaters, or the like). A dosage form of the formulations according to the present invention may be tablets, pills, granules, fine granules, capsules, or the like, and preferably tablets or granules. Examples of the capsules include hard capsules, soft capsules, and the like, and the hard capsules are particularly preferable.

The amount of the film-forming composition used may be determined according to the dosage form of a formulation. For example, when the dosage form of the formulation is tablets, the amount of the film-forming composition may be 3 to 15% by weight, relative to the weight of the formulation. To the formulation, general additives such as diluting agents, binders, lubricants, disintegrators, corrigents, preservatives, flavors, coloring agents, absorbents, humectants, disintegration retardants, or the like may be added as needed, in addition to active ingredients of drugs, cosmetics, health foods, or the like.

In the present invention, the film-forming composition may be directly included in capsules, in addition to the light-shielding agent or the film-forming composition being coated on the formulations to be contained in the capsules. In this case, the capsules have a light blocking property in themselves, as a result of which effects such that stability and quality of the inside of the capsules can be maintained for a long time, or the like, can be exerted.

Although generally known methods may be used to produce the capsules according to the present invention, one of them is shown hereinafter. That is, 15 to 30 parts by weight of hydroxypropylmethyl cellulose (HPMC) is dissolved in purified water while stirring, followed by further adding 4 to 10 parts by weight of calcium lactate to prepare a colorless and transparent immersion liquid. In this immersion liquid, stainless steel molding pins are immersed. Next, these molding pins are dried while rotating, and HPMC portions adhering to the molding pins are cut off to produce capsules.

The light-shielding agents according to the present invention include the calcium-containing compounds such as calcium salts or the like, the film-forming compositions according to the present invention include the calcium-containing compounds and the film-forming base agents exemplified by cellulose base polymers, the formulations according to the present invention are coated with the aforementioned light-shielding agents or the film-forming compositions, and the capsules according to the present invention include the aforementioned film-forming compositions.

Moreover, plasticizers, flavors, or the like may be optionally added to the film-forming composition according to the present invention, and general additives such as diluting agents or the like may be optionally added to the formulation according to the present invention.

By having such constitution, the light-shielding agents and the film-forming compositions according to the present invention, and the formulations and the capsules including either or both of these can exert effects such as inhibit separation of the surface of the formulations or powder floating phenomenon, which are caused by irradiation by light or ultraviolet light, improve luster of the surface of the formulations, maintain their external appearance for a long time and produce excellent decomposing properties, and that maintain stability and quality of pharmaceutical formulations or the like for a long time against factors such as, particularly, light, oxygen, or moisture (humidity).

Moreover, the film-forming compositions according to the present invention can be easily prepared, can prevent trouble during coating, and have excellent workablity during coating, because the film-forming compositions are different from conventional film-forming compositions containing titanium oxides, in that they are not suspensions but solutions, when they are added to solvents, and both of the calcium-containing compounds and the film-forming base agents are dissolved in solvents of the same nature (for example, both of the calcium-containing compounds and the film-forming base agents are water-soluble). Moreover, they can form white films having excellent luster without being supplemented with additives.

### Examples

In the following, the present invention will be explained in more detail by examples, but the present invention should not be interpreted to be limited to these examples.

### Test example 1

Calcium lactate was added to an aqueous solution containing 8% by weight of hydroxypropylmethyl cellulose (HPMC; manufactured by Shin-Etsu Chemical Co., Ltd.) to produce a colorless and transparent mixed solution containing 0.1 to 6% by weight of calcium lactate. This solution was casted on a glass plate, and was then dried at 60°C for 10 hours to produce films having thickness of 0.1 mm. Their film color and luster were measured by visual observation. The results are shown in Table 1. The result obtained from a solution which did not include calcium lactate is shown as a control. Calcium chloride, calcium gluconate, and magnesium chloride were respectively added to the aqueous solution containing 8% by weight of HPMC to produce respective colorless and transparent mixed solutions containing 2% by weight of each of them, from which casted films were produced in a manner similar to that above.

**Table 1**

| Concentration of added calcium lactate (relative to weight of film base agent) | Color of film | Luster |
|---|---|---|
| 0% by weight (0%) | Colorless and transparent | ++ |
| 0.1% by weight (1.25%) | White | ++ |
| 0.5% by weight (6.25%) | White | ++ |
| 1% by weight (12.5%) | White | ++ |
| 2% by weight (25%) | White | ++ |
| 3% by weight (37.5%) | White | ++ |
| 4% by weight (50%) | White | ++ |
| 5% by weight (62.5%) | White | ++ |
| 6% by weight (75%) | White | ++ |
| Luster: ++; excellent, +: good; -; no luster | | |

**Table 2**

| Kind of salt | Color of film | Luster |
|---|---|---|
| Calcium chloride | White | ++ |
| Calcium gluconate | White | ++ |
| Magnesium chloride | Colorless and transparent | ++ |
| Luster: ++; excellent, +: good; -; no luster | | |

The above results show that all of the casted films including calcium lactate, calcium chloride, or calcium gluconate have white color excellent in luster, and exert light blocking effects, while both of the casted films including magnesium chloride or HPMC alone are colorless and transparent, and do not exert light blocking effects.

### Test example 2

After 0.1 kg of polyvinyl pyrolidone and 0.1 kg of nifedipine were dissolved in 1 1 of ethanol, this solution was added to a mixture of 0.3 kg of lactose, 0.15 kg of fine crystalline cellulose, 0.4 kg of magnesium metasilicate aluminate, and 0.14 kg of carmellose calcium 0.14kg, and was then kneaded. After the kneaded material was dried at 60°C for 24 hours, and was then sifted by using a 30 mesh sieve, 8 g of magnesium stearate was added to 1 kg of the sifted material, and was then mixed, to produce nifedipine tablets having a diameter of 7 mm and a weight of 120 mg by means of a tableting machine (CLEANPRESS 19K manufactured by KIKUSUI SEISAKUSYO LTD.).

160 g of HPMC was dissolved in 1780 g of purified water, to which 40 g of calcium lactate and 20 g of polyethylene glycol (PEG6000) were further added, to produce a colorless and transparent coating solution.

This coating solution was sprayed onto nifedipine tablets by means of a doria corter (DRC-200 manufactured by Pauleck Co., Ltd.) to produce white film tablets of which film content was 8%.

Moreover, 160 g of HPMC was dissolved in 1780 g of purified water, to which 40 g of titanium oxide and 20 g of PEG6000 were further added, to produce a white suspended coating liquid. By using this liquid, white film tablets of nifedipine (its film content was 8%) were produced in a manner similar to that above. Color and luster of these films were measured by visual observation. The results are shown in Table 3.

**Table 3**

| Kind of tablet | External appearance | Luster of surface | Decomposition time (min) |
|---|---|---|---|
| Uncoated tablet | Yellow | No luster | 1 to 1.5 |
| Film tablet containing calcium lactate | White | Excellent | 3 to 3.5 |
| Film tablet containing titanium oxide | White | Bumpy | 3 to 3.5 |

The above results show that the film tablets containing calcium lactate have decomposition properties similar to those of the conventional film tablets containing titanium oxide, and have luster properties of their surface superior to those of the conventional film tablets containing titanium oxide.

### Test Example 3

The film tablets and uncoated tablets prepared in Test Example 2 were irradiated with ultraviolet light (700 mW/cm²) at constant temperature and constant humidity of 25°C-RH 50% to sequentially measure external appearance changes and nifedipine contents of the tables. The results are shown in Table 4. The nifedipine content was measured by HPLC (LC-10A manufactured by Shimazu Corporation) under the following conditions. Column: YMC-Pack ODS-A (150x4.6 mmID), Moving phase; acetonitrile: methanol: water (1:1:2), Flow rate 0.9 ml/min, Measured wavelength 254 nm.

**Table 4**

| Kind of tablet | 0th day of irradiation | | 3rd day of irradiation | | 5th day of irradiation | | 15th day of irradiation | |
|---|---|---|---|---|---|---|---|---|
| | Content | External appearance | Content | External appearance | Content | External appearance | Content | External appearance |
| Uncoated tablet | 100% | Pale yellow | 90.6% | Yellow | 86.3% | tan | 85.4% | tan |
| Calcium lactate film tablet | 100% | White | 96.7% | White (unchanged) | 94.2% | White (unchanged) | 93.0% | White (unchanged) |
| Titanium oxide film tablet | 100% | White | 92.3% | White (unchanged) | 91.4% | Unchanged | 88.3% | Occurrence of powder floating phenomena and cracks |

The results shown above demonstrate that the calcium lactate film tablets solved problems caused by the conventional titanium oxide containing film tablets, such as occurrence of powder floating phenomena and cracks on surface of the tablets, and that the content of nifedipine in the calcium lactate film tablets was satisfactorily maintained.

### INDUSTRIAL APPLICABILITY

The light-shielding agents and the film-forming compositions according to the present invention, and the formulations and the capsules including either or both of these can exert effects that prevent separation of the surface of formulations or powder floating phenomenon, which are caused by irradiation by light or ultraviolet light, improve luster of the surface of the formulations, maintain their external appearance for a long time and provide excellent decomposing properties, and that maintain stability and quality of pharmaceutical formulations for a long time against factors such as light, oxygen, or moisture (humidity).

## Claims

1. A light-shielding agent comprising a calcium-containing compound.

2. A light-shielding agent according to claim 1, wherein the calcium-containing compound is at least one selected from the group consisting of a calcium salt, a calcium hydrate, a calcium oxide, and a calcium complex.

3. A light-shielding agent according to claim 1 or 2, wherein the calcium-containing compound is at least one selected from the group consisting of calcium lactate, calcium gluconate, calcium chloride, calcium phosphate, calcium sulfate, and calcium nitrate.

4. A light-shielding agent according to any one of claims 1 to 3, wherein the calcium-containing compound is at least one selected from the group consisting of calcium lactate, calcium gluconate, and calcium chloride.

5. A film-forming composition comprising a calcium-containing compound and a film-forming base agent.

6. A film-forming composition according to claim 5, wherein the calcium-containing compound is at least one selected from the group consisting of a calcium salt, a calcium hydrate, a calcium oxide, and a calcium complex.

7. A film-forming composition according to claim 5 or 6, wherein the calcium-containing compound is the light-shielding agent according to any one of claims 1 to 4.

8. A film-forming composition according to any one of claims 5 to 7, wherein the film-forming base agent is a water-soluble agent.

9. A film-forming composition according to any one of claims 5 to 8, wherein the film-forming base agent is a saccharide.

10. A film-forming composition according to any one of claims 5 to 9, wherein the film-forming base agent is a cellulose base polymer.

11. A film-forming composition according to any one of claims 5 to 10, wherein the film-forming base agent is at least one selected from the group consisting of methylhydroxyethyl cellulose, hydroxypropyl cellulose, and hydroxypropylmethyl cellulose.

12. A film-forming composition according to any one of claims 5 to 9, wherein the film-forming base agent is a polysaccharide excepting cellulose base polymers.

13. A film-forming composition according to any one of claims 5 to 9 and 12, wherein the film-forming base agent is at least one selected from the group consisting of pullulan, carrageenan, hemilose, and alginic acid.

14. A film-forming composition according to any one of claims 5 to 13, wherein the film-forming composition includes at least one selected from the group consisting of a calcium salt, a calcium hydrate, and a calcium oxide in an amount of 0.1 to 150 % by weight, relative to a weihgt of the film-forming base agent.

15. A formulation coated with the film-forming composition according to any one of claims 5 to 14.

16. A formulation according to claim 15, wherein the formulation is a tablet, a granule, or a capsule.

17. A formulation according to claim 15 or 16, wherein the formulation is coated by a spray coating method.

18. A capsule comprising the film-forming composition according to any one of claims 5 to 14.
